(19) Europäisches Patentamt
European Patent Office
Office européen des brevets

(11) Veröffentlichungsnummer: **0 211 312 B1**

# EUROPÄISCHE PATENTSCHRIFT

(45) Veröffentlichungstag der Patentschrift: **18.12.91**

(51) Int. Cl.⁵: **A61B 10/00**

(21) Anmeldenummer: **86110035.2**

(22) Anmeldetag: **22.07.86**

Die Akte enthält technische Angaben, die nach dem Eingang der Anmeldung eingereicht wurden und die nicht in dieser Patentschrift enthalten sind.

(54) **Epikutan-Testpflaster.**

(30) Priorität: **03.08.85 DE 3527893**

(43) Veröffentlichungstag der Anmeldung: **25.02.87 Patentblatt 87/09**

(45) Bekanntmachung des Hinweises auf die Patenterteilung: **18.12.91 Patentblatt 91/51**

(84) Benannte Vertragsstaaten: **AT BE CH DE FR GB IT LI LU NL SE**

(56) Entgegenhaltungen: **EP-A- 0 026 501** **FR-A- 2 375 861**

(73) Patentinhaber: **MERCK PATENT GESELLSCHAFT MIT BESCHRÄNKTER HAFTUNG Frankfurter Strasse 250 Postfach 4119 W-6100 Darmstadt(DE)**

(72) Erfinder: **Rüdiger, Günther, Dr. Krabbenkamp 46a W-2057 Reinbek(DE)** Erfinder: **Peters, Ulf Am Steinautal 47 W-2059 Büchen(DE)**

## Beschreibung

Die Erfindung betrifft ein neues gebrauchsfertiges Epikutan-Testpflaster mit Okklusionskammern (10), das Testallergene in jeweils halbflüssigen Trägern mit definierten Dosiervolumina in den Okklusionskammern enthält, und das in einer Schutzverpackung eingelagert ist. Dieses Testpflaster kann zum Nachweis allergischer Reaktionen in der Allergiediagnostik verwendet werden.

Epikutan-Testpflaster mit Okklusionskammern sind z.B. aus EP-A-0 026 501 bekannt.

Einen besonderen Stellenwert hat in der modernen Epikutan-Testung der Finn-Chamber-Test nach V. Pirilä (vgl. H.-J. Bandmann u. S. Fregert, Epicutan-Testung, Springer Verlag, Berlin Heidelberg New York, 1982, S. 43; DE-OS 24 20 345), bei dem die Testsubstanzen vor dem eigentlichen Test manuell in die aus Aluminium bestehenden Okklusionskammern gefüllt werden.

Hierbei kommt es auf ein quantitativ äußerst genaues Füllen der Kammern an (vgl. T. Fischer u. H. Maibach, Contact Dermatitis 11 (3), Seiten 137-140 (1984)), da ein ungleichmäßiges Füllen - trotz standardisierter, industriell hergestellter Testsubstanzen - zu falsch-positiven oder flasch-negativen Testergebnissen führen kann. Diese Tatsache kann den Aussagewert von Epikutan-Testungen entscheidend beeinflussen und beeinträchtigen.

Wird zuviel Testsubstanz in eine Kammer eingebracht, so führt dies außerdem häufig auch zu einem Ablösen des Testpflasters von der Haut des Patienten.

Insbesondere gelingt es unter den Bedingungen des alltäglichen Betriebes einer Arztpraxis vielfach nicht, die Allergen-Mengen genügend genau in die Kammern zu dosieren. Außerdem nimmt das exakte Auftragen der üblichen Testsalben aus kleinen Fläschchen oder Spritzen sehr viel Zeit, Erfahrung und Geduld in Anspruch.

Der Erfindung liegt die Aufgabe zugrunde, dem Fachmann, insbesondere dem niedergelassenen, testenden Arzt, ein Testpflaster mit definierter Testfläche und moderner Kammer-Technik zur Verfügung zu stellen, das die Testallergene in exakt definierten Mengen bereits enthält, so daß es ohne zusätzlichen Aufwand sofort gebrauchsfertig ist und nach Entfernung der Schutzverpackung direkt am Patienten, in der Regel auf dem Rücken des Patienten, angelegt werden kann.

Diese Aufgabe wird durch das erfindungsgemäße Epikutan-Testpflaster gelöst.

Dieses wird hergestellt, indem man definierte Dosiervolumina der Testallergene in jeweils halbflüssigen Trägern in die Okklusionskammern füllt und das Pflaster anschließend in eine Schutzverpackung einlagert.

Vorzugsweise geht man von den bekannten "Finn-Chambers", insbesondere den "Finn-Chambers on Scanpor" (FCS) aus (vgl. l.c.). Deren Aluminiumkammern werden zweckmäßig mit einer dünnen inerten Kunststoff-Folie, z.B. aus Polytetrafluorethylen, Polypropylen, Polyetylen, ausgekleidet - wodurch Reaktionen der Aluminiumkammern mit bestimmten Testsubstanzen, z.B. Quecksilber, ausgeschlossen werden - und danach maschinell mit Gemischen, enthaltend Testsubstanzen und halbflüssige Träger, gefüllt.

Als halbflüssige Träger eignen sich die üblichen Creme-, Salben- oder Pastengrundlagen; bevorzugt ist Vaseline, aber auch Adeps lanae und Eucerin sind beispielsweise gut geeignet.

Als Testallergene werden die üblichen verwendet.

Typische Testallergene sind z.B. Mafenid, Benzocain, Procainhydrochlorid, Neomycinsulfat, Perubalsam, Formalin, Tetramethylthiuramdisulfid, 2-Mercaptobenzthiazol, Chlorjodhydroxychinolin, Terpentin, Kaliumdichromat, Nickelsulfat, Kobaltsulfat, Kolophonium, Quecksilber(II)-chlorid, Epoxidharze, p-Phenylendiamin, p-Toluylendiamin, p-Aminodiphenylaminhydrochlorid, N-Phenyl-N'-isopropyl-p-phenylendiamin, PHB-Ester (Gemisch aus p-Hydroxybenzoesäure-methyl-, -ethyl-, -propyl-, -isopropyl-, -butyl- und -benzylester), Holzteere (Gemisch aus Fichten-, Buchen-, Wacholder- und Birkenholzteer).

Bei den üblichen Finn-Chambers besitzen die Okklusionskammern einen Innendurchmesser von etwa 6-10, vorzugsweise etwa 8 mm, eine Tiefe von 0,3-1, vorzugsweise etwa 0,5 mm und ein Volumen von etwa 0,01-0,1, vorzugsweise etwa 0,025 ml. Das optimale Füllvolumen beträgt etwa 0,005 bis 0,05, vorzugsweise etwa 0,01 bis 0,02, insbesondere etwa 0,012 bis 0,015 ml.

Beim eigentlichen Abfüllvorgang ist es zweckmäßig, mit Hilfe eines Druckluftventils die erforderliche Substanzmenge bei Temperaturen zwischen etwa 20 und 50° (je nach Substanz) in die Kammern nach dem Jet-Prizip einzuschießen.

Die Temperatur wird zweckmäßig so gewählt, daß das Produkt verflüssigt, aber durch die Erwärmung nur kurz (maximal etwa 20 Sek.) thermisch belastet wird. Auf diese Weise findet praktisch keine Sedimentation statt, zumal das Füllgut hohen Beschleunigungskräften ausgesetzt wird. Unmittelbar nach der Dosierung erstarrt das Gemisch in den Kammern wieder, wobei kugelsegment- (linsen- bis halbkugel-)förmige "Salbentropfen" entstehen. Durch die kurzzeitige Verflüssigung gelingt es, die geforderten genauen Dosiervolumina einzuhalten, was bei einer Kaltdosierung sehr problematisch ist. Gleichzeitig wird bei dieser schonenden Arbeitsweise die allergische Potenz und die Qualität der Testsubstanzen nicht beeinträchtigt.

Die genaue Einstellung des Dosiervolumens und die erwünschte Kugelsegmentform des "Salbentropfens" kann gesteuert werden durch die Blocktemperatur des Ventils, den Druck, mit dem das Gemisch zugeführt wird, die Geometrie der Zuführungsdüse und die Zeit der Ventilöffnung.

Die Pflaster werden zweckmäßig in Streifen, die je 3 bis 10, zweckmäßig 5 mit Testallergenen gefüllte Kammern enthalten, konfektioniert.

Zur besseren Handhabung und Lagerung sowie zum einwandfreien Versand wird das Pflaster, z.B. in Form der genannten Streifen, in einer Schutzverpackung eingelagert, die zweckmäßig aus einer Oberfolie (5) und einer Unterfolie (11) besteht, welche das eigentliche Testpflaster sandwichartig umschließen. Als Folienmaterial eignet sich z.B. mit inertem Material, insbesondere mit weichmacher- und stabilisator-freiem Polypropylen beschichtetes Aluminium, mit einem zusätzlicher Dichtungsring (3).

Die Polypropylenschichten liegen gegeneinander; das Testpflaster ist somit hermetisch vor Umwelteinflüssen geschützt.

Zweckmäßig kann die Oberfolie außerdem jeweils über den Positionen der Testkammern halbkugelförmige Stabilisierungshauben (2) besitzen, die ein Berühren der kugelsegmentförmigen Testsubstanz-"Tropfen" und damit eine eventuelle quantitative Beeinträchtigung der auf dem Testfpflaster befindlichen genau dosierten Substanzmenge verhindert. Eine Noppenstruktur (4) auf der Oberfolie kann die Klebeflächen auf dem zwischenliegenden Testpflaster schonen und die beim Aufreißen der Schutzfolie erforderlichen Abzugskräfte verringern.

Eine besondere Ausführungsform des Pflasters besteht darin, daß es in Form eines Pfeiles zugeschnitten ist. Damit sind das obere und das untere Ende des Pflasterstreifens und damit die Lage der Testallergene auf dem Rücken des Patienten klar bezeichnet, so daß bei der Ablesung "oben" und "unten" des Testfpflasters und damit die vertikale Reihenfolge der einzelnen Testsubstanzen nicht verwechselt werden.

Bei einer weiteren Ausführung des Testpflasters ist zusätzlich eine Möglichkeit vorgesehen, verschiedene, auf dem Rücken des Patienten befindliche Testpflasterstreifen auch qualitativ optisch voneinander differenzieren zu können.

Diese zusätzliche optische Kennzeichnung ist wichtig, wenn z.B. nach Ablauf von 24-48 Stunden die verschiedenen Testpflaster vom Rücken des Patienten entfernt und ca. 30 min später die jeweiligen Hautreaktionen vom Arzt abgelesen werden.

Zur eindeutigen Identifikation der jeweiligen (negativen oder positiven) Testreaktionen auf der Haut des Patienten werden die Art und die Reihenfolge der Substanzen jedes Teststreifens üblicherweise mit einem speziellen Hautmarkierungsstift direkt auf die Rückenhaut des Patienten notiert, so daß die exakte Zuordnung der Hautreaktionen zu den getesteten Substanzen ermöglicht wird.

Diese Art der Hautmarkierung zur eindeutigen Wiedererkennung und Identifikation der Testreaktionen ist sehr zeitaufwendig und belästigt den Patienten durch mögliche Verfärbungen. Sie kann wie folgt vermieden werden.

Eine z.B. am oberen Rand des Pflasters angebrachte Solltrennlinie oder Perforation erlaubt, daß nach Ablauf der Testzeit der untere Teil des Pflasterstreifens mit den Testkammern entfernt werden kann, der oberhalb der Solltrennlinie (Perforation) sitzende kleinere Pflasterabschnitt jedoch auf dem Rücken des Patienten verbleibt. Dieser kleine Pflasterabschnitt kann herstellerseitig bereits mit einer Kodierzahl oder einem Kodierbuchstaben versehen sein, der auf eine festgelegte Reihenfolge bestimmter Testsubstanzen verweist.

So könnte ein Teststreifen mit der römischen Ziffer I bezeichnet sein und beispielsweise die fünf Testallergene in definierter Reihenfolge und Testkonzentration bezeichnen und festlegen:

| Beispiel Standard-Reihe "I" (von oben nach unten): | |
|---|---|
| Kaliumdichromat | 0,5% |
| Benzocain | 5 % |
| Tetramethylthiuramdisulfat | 1 % |
| Formaldehyd | 1 % |
| Adeps Lanae | 30 % |

(Dies sind die ersten fünf Testallergene einer aus 20 wichtigen Kontaktallergenen bestehenden Reihe des "Westdeutschen Standards").

Die Anwendung des neuen Testpflasters ist einfach. Der Anwender, z.B. der Arzt oder die Arzthelferin, braucht die Schutzverpackung nur noch aufzureißen und die Pflasterstreifen anzulegen. Das bisher übliche zeitaufwendige und mühsame Füllen der Testkammern unter dem Zeitdruck des Praxisbetriebes und daraus sich ergebende unregelmäßige Füllmengen und Testergebnisse entfallen.

Mit Hilfe des neuen Testpflasters sind kontrollierte Aussagewerte, größere Genauigkeit und erhöhte Zuverlässigkeit der Testungen und späterer Kontrolltestungen ermöglicht. Da die Testallergene stets in gleichmäßiger Qualität, genauer Dosierung und zweckmäßig in einer mit inertem Material ausgekleideten Okklusionskammer aufgebracht sind, wird die Gefahr falsch-negativer bzw. falsch-positiver Reaktionen vermindert. Bei der Anwendung wird gegenüber der üblichen Praxis Zeit gespart, da kein mühsames manuelles Füllen der Kammern im Allergielabor mehr nötig ist. Schließlich kann mit Hilfe des neuen Testpflasters dem niedergelassenen, testenden Arzt eine große Anzahl auch ungewöhnlicher Testallergene zur Verfügung gestellt werden, denn der Versand und die Lagerung der Testpflaster ist durch die beschriebene Schutzverpackung problemlos geworden.

Ein bevorzugtes Ausführungsbeispiel ist in der Zeichnung dargestellt.

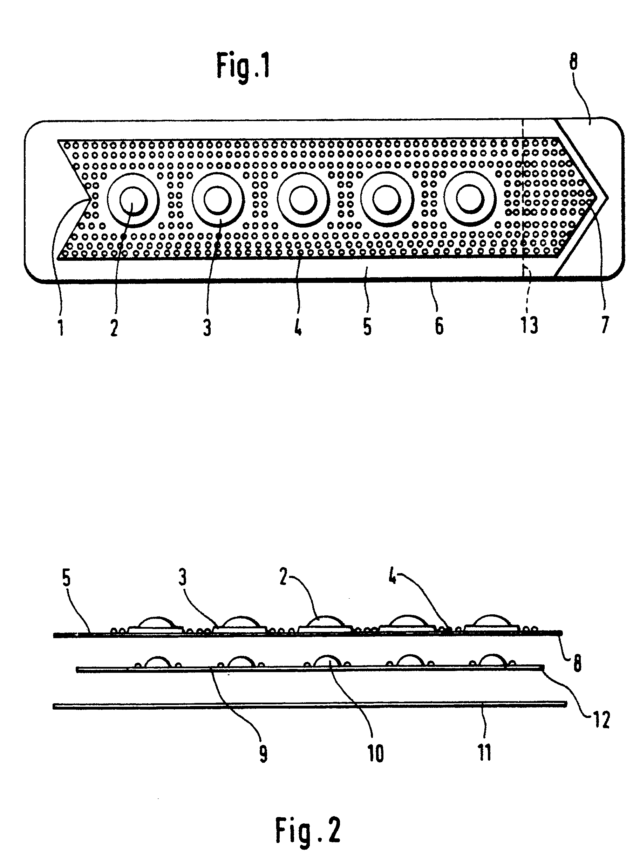

Fig. 1    Draufsicht (Oberfolie)
Fig. 2    Seitenansicht (Fliehteilzeichnung von Oberfolie, FCS-Pflaster und Unterfolie)
1.    negativ gepfeiltes Ende der Oberfolie
2.    Stabilisierungshaube über der Aluminiumkammer
3.    Dichtungsring über der Aluminiumkammer
4.    Abstandsnoppen zum eigentlichen Pflaster (siehe Fig. 2)
5.    Oberfolie (nicht-genoppter Rand)
6.    umlaufender Siegelrand (verbindet Ober- u. Unterfolie)
7.    positiv gepfeiltes Ende der Oberfolie
8.    Abreißlasche zur Trennung von Ober- u. Unterfolie
9.    FCS-Pflaster
10.    Aluminiumkammer (Finn-Chamber) mit Testallergenen
11.    Unterfolie
12.    gepfeiltes Ende des Pflasters
13.    Solltrennlinie (Perforation)

## Patentansprüche

1. Gebrauchsfertiges Epikutan-Testpflaster mit Okklusionskammern (10) aus Aluminium, die mit einer dünnen inerten Kunststoff-Folie ausgekleidet sind, wobei die Okklusionskammern Testallergene in jeweils halbflüssigen Trägern mit definierten Dosiervolumina in Form von kugelsegmentförmigen Testsubstanz-"Tropfen" enthalten, und das Testpflaster in einer aus einer Oberfolie (5), die jeweils über den Positionen der Okklusionskammern halbkugelförmige Stabilisierungshauben (2) und Dichtungsringe (3) besitzt, und einer Unterfolie (11) bestehenden Schutzverpackung eingelagert ist.

2. Verfahren zur Herstellung eines Epikutan-Testpflasters mit Okklusionskammern gemäß Patentanspruch 1, dadurch gekennzeichnet, daß man definierte Dosiervolumina der Testallergene in jeweils halbflüssigen Trägern kurz thermisch belastet, das so verflüssigte Produkt in die Okklusionskammern füllt, wo es unter Entstehung kugelsegmentförmiger "Testsubstanztropfen" wieder erstarrt, und das Pflaster anschließend in die Schutzverpackung einlagert.

## Claims

1. Plaster which is ready for use for patch skin tests and which has occlusion chambers (10) which are composed of aluminium and which are lined with a thin film of inert plastic, where the occlusion chambers contain test allergens, each in semiliquid vehicles with doses of defined volume in the form of test substance "drops" in the form of segments of a sphere, and the test plaster is enveloped in a protective pack which consists of an upper foil (5), which has, in each case over the positions of the occlusion chambers, hemispherical stabilizing convexities (2) and gaskets (3), and of a lower foil (11).

2. Process for the preparation of a plaster for patch skin tests with occlusion chambers according to Patent Claim 1, characterised in that doses of defined volume of the test allergens, each in semiliquid vehicles, are briefly exposed to heat, the product which has been liquefied in this way is introduced into the occlusion chambers, where it solidifies again to result in "test substance drops" in the form of segments of a sphere, and the plaster is then enveloped in the protective pack.

**Revendications**

1.  Emplâtre d'essai épicutané prêt à l'emploi, avec des chambres d'occlusion (10) en aluminium qui sont revêtues d'une feuille mince et inerte de matière plastique, dans lequel les chambres d'occlusion contiennent des allergènes d'essai dans des supports semi-liquides en volumes de dosage bien définis sous forme de "gouttes" de substance d'essai en forme de segments de sphères et en ce que l'emplâtre d'essai est placé dans un emballage de protection constitué d'une feuille supérieure (5) présentant des coiffes de stabilisation hémisphériques (2) et des bagues d'étanchéité (3) au-dessus des emplacements des chambres d'occlusion,et d'une feuille inférieure (11).

2.  Procédé de fabrication d'un emplâtre d'essai épicutané avec des chambres d'occlusion selon la revendication 1, caractérisé en ce qu'on sollicite thermiquement, pendant une brève période, des volumes de dosage bien définis des allergènes d'essai dans des supports semi-liquides et que le produit ainsi liquéfié est introduit dans les chambres d'occlusion où il se solidifie à nouveau avec formation de "gouttes de substance d'essai" en forme de segments de sphères et que l'emplâtre est ensuite placé dans l'emballage de protection.

# Fig.1

# Fig.2